# EUROPÄISCHE PATENTSCHRIFT

(11) **EP 0 476 004 B1**
(45) Veröffentlichungstag und Bekanntmachung des Hinweises auf die Patenterteilung: **26.05.1993**
(21) Anmeldenummer: 90908915.3
(22) Anmeldetag: 06.06.1990
(51) Int. Cl.: H05B 6/80

(54) **VERFAHREN UND VORRICHTUNG ZUR EINSTRAHLUNG VON MIKROWELLENENERGIE IN WASSERHALTIGE ODER MIT WASSER VERSETZTE MATERIE**
PROCESS AND DEVICE FOR IRRADIATING MATERIAL CONTAINING OR MIXED WITH WATER WITH MICROWAVE ENERGY
PROCEDE ET DISPOSITIF POUR IRRADIER AVEC DES MICRO-ONDES DES MATIERES CONTENANT DE L'EAU OU MELANGEES A DE L'EAU

(30) Priorität: 07.06.1989 AT 1403/89
(43) Veröffentlichungstag der Anmeldung: 25.03.1992
(73) Patentinhaber: MOSHAMMER, Wolfgang, Dipl.-Ing., A-8010 Graz (AT); FREISSMUTH, Johann, A-8010 Graz (AT)
(72) Erfinder: MOSHAMMER, Wolfgang, Dipl.-Ing., A-8010 Graz (AT); BERGER, Erwin, A-8750 Judenburg (AT); FREISSMUTH, Johann, A-8010 Graz (AT); FREIBERGER, Helmut, A-1120 Wien (AT)
(74) Vertreter: Krause, Walter, Dr. Dipl.-Ing.
(86) Internationale Anmeldenummer: AT9000057
(87) Internationale Veröffentlichungsnummer: WO9015515

(56) Entgegenhaltungen:
- EP-A- 039 517
- EP-A- 112 295
- EP-A- 0 016 385
- FR-A- 2 359 633
- FR-A- 2 612 033
- GB-A- 2 132 325
- US-A- 4 313 786
- US-A- 4 606 650
- US-A- 4 715 727

## Beschreibung

Die Erfindung betrifft eine Vorrichtung zur Einstrahlung von Mikrowellenenergie in wasserhältige oder mit Wasser versetzte Materie, mit einem Hohlraumresonator, welcher mit zumindest einem, in seiner Energieabgabe regelbaren Mikrowellen-generator in Verbindung steht, mit einem für Mikrowellen durchlässigen, gasdicht verschließbaren Gefäß zur Aufnahme der zu behandelnden Materie, dessen Innenraum der Hohlraumresonator mit Abstand umschließt, wobei der Innenraum des Gefäßes mit einer Druckmesseinrichtung in Verbindung steht und eine die Energieabgabe des Mikrowellengenerators beeinflussende, mit der Druckmeßeinrichtung in Verbindung stehende Regeleirichtung vorgesehen ist. Eine derartige Vorrichtung ist beispielsweise aus Druckschrift US-A-4 606 650 bekannt. Weiters betrifft die Erfindung ein Verfahren zur Einstrahlung von Mikrowellenenergie in wasserhältige oder mit Wasser versetzte Materie.

Es sind eine Reihe von Anwendungsgebieten bekannt geworden, wo Mikrowellen zum Erwärmen bzw. Erhitzen von vor allem wasserhältiger, organischer Materie verwendet werden. Die Produktvielfalt reicht von Geräten zum Kochen, Garen und Braten bis zu Vorrichtungen zum Sterilisieren, Desinfizieren oder Dehydratisieren von Gegenständen bzw. Proben.

So ist beispielsweise aus der EP-A 0 287 549 eine Vorrichtung bekannt geworden, welche zum Erhitzen von Gegenständen und Organismen, insbesondere zum Abtöten bzw. Inaktivieren von eiweiß- bzw. nukleinsäurehältigen Organismen dient. Dabei werden die in einen Hohlraumresonator einbringbaren Gegenstände bzw. Organismen von mittels Magnetrons erzeugten Mikrowellen beaufschlagt. Zur homogenen Energieverteilung bzw. zur Vermeidung sogenannter kalter Stellen im Hohlraumresonator sind mehrere Magnetrons in bestimmter geometrischer Anordnung vorgesehen, wodurch dieses Gerät besonders für die Sterilisation medizini-scher Geräte und für die Entsorgung medizinischen (infektiösen) Abfalls geeignet sein soll.

Nachteilig bei diesem bekannten Gerät ist vor allem, daß die in organischer bzw. befeuchteter Materie erreichbaren Temperaturen von 100°C nicht ausreichen um beispielsweise Sporen abzutöten, wozu Temperaturen ≧ 121°C notwendig wären.

Als weiterer Nachteil ist anzuführen, daß Trägermaterialien durch direkte Mikrowellenanregung auf weit höhere Temperaturen erhitzt werden können, wodurch zum Teil die Zündtemperatur von Papier- und Stoffteilen erreicht und überschritten werden kann.

Diese Probleme können auch durch die im Inneren des Resonators angeordneten Wasserbeutel nicht gelöst werden. Die für eine wirksame Durchfeuchtung der einhgebrachten Proben bzw. wirksamen Kühlung von direkt durch Mikrowellen anregbaren Trägermaterialien notwendige homogene Verteilung des Wasserdampfes kann dadurch nicht erreicht werden.

Des weiteren ist es mit dem genannten Gerät bzw. auch mit anderen bekannten Mikrowellengeräten zum Erwärmen oder Erhitzen von organischer Materie nicht möglich, das Austrocknen der in den Hohlraumresonator einbringbaren Materialien zu unterbinden, was insbesondere beim Zubereiten oder Wärmen von Speisen von Nachteil ist.

Zum Sterilisieren bzw. Desinfizieren sind weiters Autoklaven bekanntgeworden. Sie beruhen auf dem Prinzip, daß in einen gegebenenfalls vorher evakuierten Druckbehälter Dampf mit einer definierten Temperatur eingelassen wird. Dieser Dampf enthält eine bestimmte Wärmeenergie, die an die eingebrachte Materie abgegeben wird. Aufgrund des Temperaturunterschiedes von Wasserdampf und Materie kommt es zu einen von den Wärmeleiteigenschaften des Materials abhängigen Wärmetransport. Durch diesen Wärmetransport kühlt der eingebrachte Wasserdampf ab, sodaß zur Erreichung einer vorgebbaren Temperatur der Druckbehälter erneut evakuiert und wieder mit Heißdampf gefüllt werden muß. Um möglichst rasch eine bestimmte Temperatur zu erreichen, muß der Wasserdampf erheblich heißer in das Druckgefäß eingebracht werden, als die gewünschte Temperatur der Materie betragen soll. Das wiederum bedeutet einen zusätzlichen Aufwand an Energie. Zudem muß der Wasserdampf auch die Wände des Druckbehälters miterwärmen. Auch diese Energie geht letztendlich nutzlos verloren und leistet keinen Beitrag zur Erwärmung der Materie. Liegt Materie mit schlechten Wärmeleiteigenschaften und großem Volumen bei gleichzeitig geringer Oberfläche vor, so dauert es sehr lange, bis der Wärmetransport vom Wasserdampf bis zum Inneren der Materie erfolgt, wobei in geschlossenen Innenräumen, beispielsweise in Kunststoffschläuchen, oft trotzdem ein Überleben von Keimen möglich ist. Nachteilig sind auch die langen Zykluszeiten von 50 Minuten und mehr. Die gesamte aufgewendete Energie beträgt somit letztlich ein Vielfaches jener Energie, die im zu erwärmenden Gut steckt.

Aus der DE-Al 33 41 585 ist es bekannt, das zu erwärmende Objekt in einen druckdichten, metallisch begrenzten Arbeitsraum, also in den eigentlichen Resonator einzubringen, wobei das zu erwärmende Objekt mit einer Schweißfolie vakuumverpakt ist. Da die Schweißfolie den gewünschen Drücken bis zu mehreren bar nicht standhalten kann, kommt es zu einem Anliegen der Folie an Teilen der Resonatorwand.

Die Resonatorwand ist jedoch bekanntermaßen jene Grenzfläche, an welcher die elektrische Feldstärke der Mikrowelle gleich Null sein muß, wodurch es an der Resonatorinnenoberfläche auch zu keiner Erwärmung des eingebrachten Gutes bzw. des vorliegenden Dampfes kommt. Aufgrund des Kühleffektes an der Resonatoroberfläche kommt es hier im Gegenteil zu einher Abkühlung des Dampfes. Der Dampf kondensiert also an der Resonatoroberfläche und sammelt sich am Boden. Die entsprechende Flüssigkeit ist somit nicht den Sterilisationsbedingungen ausgesetzt und müßte daher auch nach dem Sterilisationsvorgang als infektiös angesehen werden.

Da beispielsweise infektiöser Abfall nahezu ausschließlich in eigens dafür vorgesehenen Säcken in den Mikrowellendesinfektor gelangt, und der Abfall als schüttbares Gut anzusehen ist, würde das aufgezeigte Problem auch beim Abfall selbst auftreten. Abfallteile, welche an der Innenwand des Resonators zu liegen kämen, blieben ebenfalls infektiös.

Aus der US-PS 4 606 650 ist eine Vorrichtung der eingangs genannten Art bekannt, mit welcher im Vakuum wäßrige Proben getrocknet werden können. Zu diesem Zweck befindet sich im Hohlraumresonator ein Gefäß, welches die Proben aufnimmt und über eine Durchführung durch eine Dichtung zwischen einem Unter-und einem Oberteil des Gefäßes mit einer Vakuumleitung in Verbindung steht. Eine Kontrolleinheit steht sowohl mit einer Druckmeßeinrichtung als auch mit einer Steuereinrichtung zur Steuerung des Mikrowellengenerators in Verbindung. Das Gefäß dieses bekannten Mikrowellenofens kann jedoch keine Uberdruckkräfte aufnehmen, wie dies bei der Desinfektion von Proben notwendig wäre. Ein ähnlich arbeitendes Mikrowellengerät ist weiters aus der GB-A 2 132 325 bekannt.

Aufgabe der Erfindung ist es, ein Verfahren bzw. eine Vorrichtung zur Einstrahlung von Mikrowellenenergie in wasserhältige oder mit Wasser versetzte Materie vorzuschlagen, mit welcher eine möglichst homogene Erwärmung bzw. Erhitzung auch sehr inhomogener Materie möglich ist. Für die Sterilisation medizinischer Geräte und die Behandlung medizinischer, gegebenenfalls infektiöser Abfälle sollen auch Temperaturen zum Abtöten von Sporen erreicht bzw. überschritten werden.

Diese Aufgabe wird erfindungsgemäß dadurch gelöst, daß sich das gasdicht verschließbare Gefäß am Hohlraumresonator abstützt und als Verkleidung des Hohlraumresonators ausgebildet ist, daß eine mit einem Ventil versehene Zuleitung für Wasser oder Wasserdampf mit dem Innenraum des Gefäßes in Verbindung steht, sowie daß die zur Erreichung und Konstanthaltung eines vorgebbaren, über dem Atmosphärendruck liegenden Innendruckes benötigte Energieabgabe des Mikrowellengenerators über die Einrichtung regelbar ist. Durch die Einrichtung zur Regelung und Konstanthaltung eines vorgebbaren Innendruckes kann die im Inneren des gasdicht verschließbaren, druckfesten Gefäßes vorherrschende Temperatur in die für die Sterilisation notwendigen Bereiche angehoben erden. Im Inneren des geschlossenen Gefäßes entsteht rasch ein relativ hoher Wasserdampfgehalt, welcher für den geforderten Temperaturausgleich und eine homogene Erwärmung der eingebrachten Materie sorgt. Materie mit zu geringem Wassergehalt kann Wasser oder Wasserdampf direkt zugesetzt werden. Die Druckmessung kann direkt über einen entsprechenden Sensor oder indirekt über eine mit dem Innenraum des Gefäßes in Verbindung stehende Temperaturmeßeinrichtung erfolgen.

Die Wände des gasdichten Gefäßes, welche als Verkleidung oder Beschichtung des Resonators ausgebildet sein können, sorgen für eine thermische Isolierung des Innenraumes vom Hohlraumresonator, wobei dessen Wandstärke den nötigen Abstand des Innenraumes von der Wand des Hohlraumresonators bestimmt, welcher für eine Mikrowellenfrequenz von 2450 MHz im Bereich von 3 cm liegt. Das Gefäß muß eine Dichtfunktion erfüllen, sowie geringe Hochfrequenzverluste und eine hohe Temperaturbeständigkeit aufweisen. Die entstehenden Verformungskräfte werden zum Großteil vom Hohlraumresonator aufgenommen.

Das als Verkleidung des Hohlraumresonators ausgebildete Gefäß kann beispielsweise aus Polytetrafluorethylen oder aus Silikon bestehen.

Ein erfirdungsgemäßes Verfahren zur Einstrahlurg von Mikrowellenenergie in wasserhältige oder mit Wasser versetzte Materie ist durch folgende Schritte gekennzeichnet:
a) Einbringen der zu behandelnden Materie in einen gasdichten, druckfesten, für Mikrowellenstrahlung durchlässigen Behälter,
b) Evakuieren des gasdichten Behälters,
c) Zusetzen von Wasser oder Wasserdampf in Abhängigkeit vom Wassergehalt der zu behandelnden Materie,
d) Einstrahlen von Mikrowellenenergie zur Erhitzung der Materie und des zugesetzten Wassers bzw. Wasserdampfes auf Temperaturen über 100°C und Erhöhung des Innendruckes auf einen vorgebbaren Druck über dem Atmosphärendruck,
e) Messen des Innendruckes,
f) Regeln der Mikrowelleneinstrahlung zur Erreichung und Konstanthaltung des vorgebbaren Innendruckes in Abhängigkeit des in Schritt e) erzielten Meßwertes und
g) Aufrechterhalten des in Schritt f) erreichten Innendruckes für einen vorgebbaren Zeitintervall um einen Temperaturausgleich zwischen den Materiebestandteilen zu erreichen.

Insbesondere ist es von Vorteil, wenn die Materie vor der Einkopplung der Mikrowellenenergie einem unter dem Atmosphärendruck liegenden Absolutdruck, vorzugsweise ca. 100 mbar, ausgesetzt wird, wobei eine mit einem Ventil abtrennbare, außerhalb des Hohlraumresonators angeordnete Vakuumeinrichtung zur Evakuierung des Innenraumes vorgesehen ist. Dadurch wird der Wasserdampfgehalt im Reaktionsraum weiter erhöht, wodurch eine raschere Erhitzung sowie ein schnellerer Temperaturausgleich zwischen inhomogenen Materiebestandteilen im verschließbaren Innengefäß ermöglicht wird.

Schließlich ist erfindungsgemäß vorgesehen, daß das Gefäß und der Hohlraumresonator über einen gemeinsamen Deckel verfügen, welcher einen Verriegelungsmechanismus mit einer Kontroll- und Sicherheitseinrichtung aufweist.

Die Erfindung wird im folgenden anhand einer schematischen Zeichnung näher erläutert.

Die dargestellte Vorrichtung, welche zum Sterilisieren bzw. Desinfizieren von Materie, vorzugsweise zur Desinfektion infektiöser Abfälle aus dem medizinischen Bereich dient, weist ein gasdicht verschließbares Gefäß 1 zur Aufnahme der zu behandelnden Materie auf, welches den Hohlraumresonator 2 aus-kleidet. Der Mindestabstand des Innenraumes 3 von der Resona--torwand liegt für Mikrowellen von 2450 MHz im Bereich von 3 cm. Mit dem Innenraum 3 steht eine Druckmeßeinrichtung 4 in Verbindung, welche über eine Steuerelektronik 5 die Energieabgabe eines Mikrowellengenerators 6 steuert. Es wäre jedoch auch möglich, den Innendruck über eine Temperaturmessung zu erfassen. Weiters steht mit dem Gefäß 1 eine Vakuummeinrichtung 7 zur Evakuierung des Innenraumes 3 in Verbindung. Die Vakuumeinrichtung 7 weist eine von der Druckmeßeinrichtung 4 über die Steuerelektronik 5 steuerbare Vakuumpumpe 8 sowie ein Ventil 9 zur Aufrechterhaltung des Innendruckes auf. Die mit der Druckmeßeinrichtung 4 verbundene Steuerelektronik 5 sowie die Vakuumeinrichtung 7 bilden zusammen mit den entsprechenden Steuerleitungen die Einrichtung 10 zur Regelung und Konstanthaltung eines vom atmosphärischen Druck abweichenden Innendruckes. Eine Zuleitung 11 mit einem Dosierventil 12 ist für die Zufuhr von Wasser bzw. Wasserdampf vorgesehen, um zu trockene Materie entsprechend anzufeuchten.

Beim Betrieb als Sterilisator sind folgende Punkte zu beachten:
In das Gefäß 1 wird die zu bestrahlende Materie eingebracht und mittels des Dosierventiles 12 händisch oder auch automatisch Wasser eingebracht, wobei dieser Punkt entfallen kann, falls die Materie selbst wasserhältig ist. Anschließend wird das Gefäß 1 gasdicht und druckfest verschlossen. Für besondere Anwendungsfälle, beispielsweise zur Desinfektion, kann das Druckgefäß vor Einstrahlung der Mikrowellenenergie Dis auf etwa 100 mbar evakuiert werden, sodaß beim nachfolgenden Erhitzen gesättigter Wasserdampf entsteht. Nach Beendigung des Evakuiervorganges wird das Drucksystem sich selbst überlassen und die Vakuumeinrichtung 7 abgeschaltet. In den Hohlraumresonator 2 wird nun z.B. mittels Gyratron, Magnetron, Klystron oder andere Mikrowellengeneratoren 6 Mikrowellenenergie eingekoppelt, welche die eingebrachte Materie erwärmt, sofern sie durch Mikrowellenenrgie direkt erwärmbar ist, bzw. das zugesetzte Wasser erhitzt. Aufgrund des verdampfenden Wassers steigt der Druck im Innenraum 3 des Gefäßes 1, wodurch auch die Siedetemperatur des Wassers auf Werte über 100°C ansteigt. Über die Druckmeßeinrichtung 4 wird der Mikrowellengenerator 6 durch Drosselung bzw. zeitweiliges Ausschalten derart gesteuert, daß ein vorgebbarer Druckwert (z.B. 3 bar Absolutdruck für Sterilisation und 2 bar Absolutdruck für Desinfektion) nicht überschritten wird. Der entstehende Wasserdampf erfüllt das gesamte Drucksystem und stellt somit ein gleichmäßiges Temperaturfeld dar und wird überdies vom Mikrowellenfeld ständig auf einer konstanten Temperatur gehalten. Der Wärmeumsatz entsteht im Wasserdampf selbst. Werden Teile der eingebrachten Materie durch Mikrowellenenergie nicht erwärmt, so wird Wärme vom Wasserdampf zu diesen Materiebestandteilen geleitet. Die Folge davon ist eine allmähliche Erwärmung der gesamten Materie auf die Temperatur des Wasserdampfes. Werden Teile der eingebrachten Materie durch Mikrowellenenergie stärker erwärmt als das umgebende Wasser, so wird Wärme von der Materie zum Wasser bzw. Wasserdampf geleitet. Die Folge ist eine Kühlung dieser Materieteile von der Oberfläche her, wodurch diese Materieteile (Trägermaterialien, Operationsbesteck, Küvetten) trotz Energiezufuhr nicht beliebige Temperaturen annehmen können. Nach einer vorgebbaren Betriebsdauer ist festzustellen, daß sowohl die eingebrachte Materie als auch der Wasserdampf aufgrund des Wärmeaustausches annähernd dieselbe Temperatur aufweisen, was bedeutet, daß über die Messung des Druckes oder auch der Temperatur des Wasserdampfes eine Aussage über die Temperatur der eingebrachten Materie gemacht werden kann. Während des gesamten Betriebes wird die Zufuhr der Mikrowellenenergie so gedrosselt oder unterbunden, sodaß ein vorgegebener Maxinaldruck nicht überschritten, aber auch ein Minimaldruck nicht unterschritten wird, wobei die gesamte Materie eine vorbestimmbare Zeit einer vorbestimmbaren Temperatur ausgesetzt ist. Die Mikrowellenzufuhr wird nach einer vorgesehenen Betriebszeit unterbrochen, der Druck im Gefäß 1 wird abgelassen und die Materie entnommen.

Das Gefäß 1 kanm bei größeren und großen Ausführungsformen, beispielsweise für die Abfalldesinfektion, aus einem Material bestehen, mit welchem eine thermische Isolierung vom Hohlraumresonator 2 möglich ist. Das hat den erheblichen Vorteil, daß beim Erwärmen der Materie der Hohlraumresonator nicht miterwärmt werden muß, was sich in einer erheblichen Energie- und Zeitersparnis bemerkbar macht.

Schematisch ist ein gemeinsamer Deckel 13 des Gefässes 1 und des Hohlraumresonators 2 in Offenstellung angedeutet. Das Gefäß 1 muß während des Desinfektionszyklusses gegen Öffnen gesichert sein. Dazu wird zu Beginn des Desinfekt-ionsvorganges der Deckel 13 mit Hilfe des Verriegelungsantriebes 14 automatisch verriegelt. Der zugeordnete Verriegelungskontakt 15 kontrolliert den Verriegelungsmechanismus. Aus Sicherheitsgründen sind für das sichere Verschließen des Deckels 13 zwei Endschalter 16, 17 vorgesehen, wovon einer 16 mit der Steuerelektronik 5 in Verbindung steht und der andere 17 in einen eigenen, hier nicht dargestellten, unabhängigen Steuerkreis eingebunden ist. Der Deckel 13 weist auch eine Streustrahlungsüberwachung 18 auf, welche die vom Gesetzgeber maximal zulässige Leistungsdichte der austretenden Strahlung überwacht. Um eine Kontrolle über lange Zeit zu ermöglichen, ist es sinnvoll, die Streustrahlung während des Betriebes fortlaufend zu messen und mit einem vorgegebenen Schwellwert zu vergleichen. Kommt es zu Überschreitungen, wird dies von der Elektronik registriert und das Gerät abgeschaltet.

Die Einbringung der benötigten, vorzugsweise entkalkten Wassermenge um den gesamten Innenraum 3 des Gefäßes 1 bei Nenndruck mit Wasserdampf zu füllen, erfolgt über eine Sprühdüse 19, welche das über die Zuleitung 11 zugeführte Wasser in kleine Tröpfchen zerstäubt, um den Energieumsatz der eingestrahlten Mikrowellen im Wasser zu vergrößern. In der Zuleitung 11 befindet sich außer dem Dosierventil 12 eine Leitungsdrucküberwachung 20 sowie ein Druckminderventil 21, zur Bereitstellung eines konstanten Betriebsdruckes.

Die Vakuumpumpe 8 erzeugt im Gefäß 1 vor der Einkoppelung der Mikrowellen einen Absolutdruck von ca. 100 mbar. Dadurch entsteht in der nachfolgenden Druckphase ein Luft-Dampf-Gemisch mit einem sehr hohen Anteil an Wasserdampf. Durch die hohe spezifische Wärme des Wassers wird mittels Dampf viel Wärmenergie an das Desinfektionsgut von außen herangebracht, wobei auch kleinen Hohlräumen optimal Wärmenergie zugeführt wird. Sobald der gewünschte Unterdruck erzeugt ist, wird dieser auch nach dem Abschalten der Vakuumpumpe 8 durch das Ventil 9 aufrecht erhalten. Weiters muß in der Phase des Überdruckes die Vakuumpumpe 8 vor dem Druck im Innenraum 3 geschützt werden. Das Evakuieren des Gefäßes 1 am Beginn des Desinfektionsvorganges erfolgt über die Verbindungsleitung 22 und die Sprühdüse 19 der Einspritzvorrichtung, weil dadurch die Gefahr der Ausbringung von Keimen am geringsten ist. Dabei ist das Druckhalteventil 23 in der Leitung 11 bis nach der Einspritzphase geöffnet. Während der Druckphase im Gefäß 1 dichtet das Druckhalteventil 23 das Gefäß 1 nach außen ab. Zum Belüften des Gefäßes 1 nach denn letzten Evakuiervorgang wird Außenluft über das Vakuumventil 24 in das Gefäß 1 eingelassen. Nach erfolgter Desinfektion und Druckausgleich im Gefäß 1 entsteht im Innenraum 3 kondensiertes Wasser. Dieses ebenfalls keimfreie Wasser wird nach dem Öffnen des Abwasserventiles 25 mitels Vakuumpumpe 8 über die Pumpleitung 26 aus dem Gefäß 1 gepumpt. Die abgepumpte Luft aus dem Evakuierungsvorgang sowie das Kondensat gelangen in den Abfluß 27.

Da nicht ausgeschlossen werden kann, daß der Drucksensor der Meßeinrichtung 4 aufgrund eines Defektes falsche Daten an die Steuerelektronik 5 meldet, erfolgt eine weitere unabhängige Druckerfassung. Dies geschieht durch die Druckschalter 28 und 29, die nur ein Digitalsignal abgeben. Der Schaltdruck des Druckschalters 28 liegt unter dem Nenndruck, d.h., bei Nenndruck ist der Druckschalter 28 eingeschaltet. Sollte der Druckschalter 28 bei Nenndruck nicht ansprechen, so stimmt die Druckmessung zwischen Meßeinrichtung 4 und den Druckschaltern nicht überein, womit der Desinfektionsvorgang von der Steuerelektronik abgebrochen wird. Der Schaltdruck des Druckschalters 29 liegt über dem Nenndruck. Das bedeutet, daß Druckschalter 29 bei Nenndruck ausgeschaltet ist. Sollte der Druckschalter 29 ansprechen, wird der Desinfektionsvorgang ebenfalls abgebrochen. Um bei Ausfall der Elektronik unerwünschte Druckanstiege im Gefäß 1 zu vermeiden, ist ein mechanisches Überdruckventil 30 vorgesehen.

Verbindungsleitungen der einzelnen elektromagnetisch bzw. elektromechanisch betätigbaren Ventile sowie der Endschalter 16, 17 und der Druckschalter 28, 29 sind nicht dargestellt.

Um ein Überhitzen der Mikrowellengeneratoren 6 zu vermeiden, sind diese jeweils mit Kühlventilatoren 31 ausgestattet.

Die Steuerelektronik 5 weist ein Tastenfeld mit einer Start-, einer Stop- und einer ID-Taste zur Identifizierung von Fehlerzuständen in einem nicht korrekt ablaufenden Desinfektionszyklus auf.

Ein alphanumerisches Display 32 zeigt die für den Benutzer relevanten Betriebsinformationen, sowie dient zur Anzeige von internen Betriebsparametern für den Servicetechniker. Weiters ist für Zwecke der Dokumentation ein Drucker 33 zum Ausdrucken der wesentlichen Betriebsparameter (Datum, Uhrzeit, Vakuum, Nenndruck, Desinfektionszeit) vorgesehen. Fehlerhafte Betriebszustände körnen zusätzlich von einer akustischen Signaleinrichtung 34 angezeigt werden.

Die mit Steuerelektronik 5 zusammengefaßte Einheit weist eine Steuerungs- und Überwachungseinheit auf der Basis eines Mikroprozessors, einen Netzteil und die Leistungselektronik, sowie die für das sichere Funktionieren notwendige Elektronik auf.

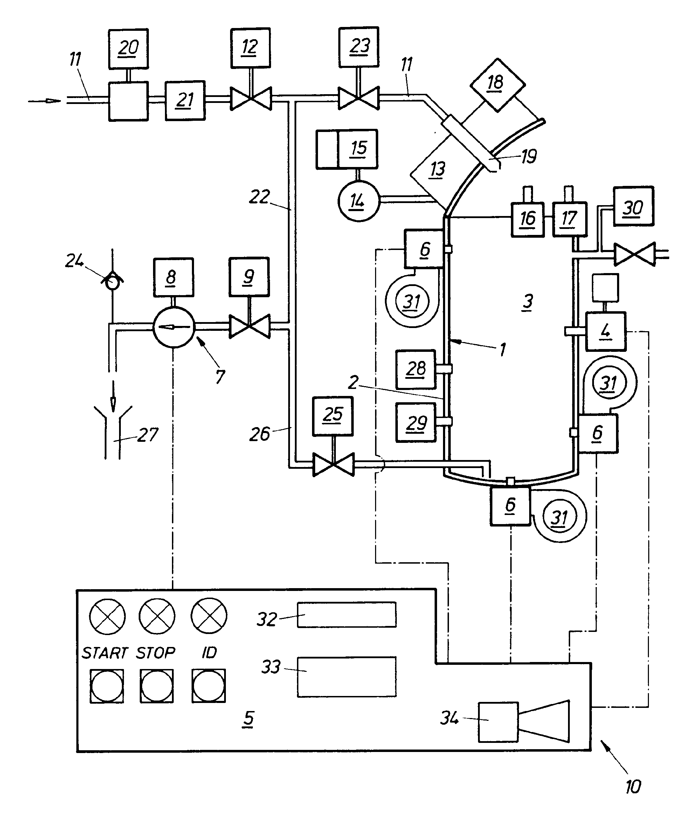

## Patentansprüche

1. Vorrichtung zur Einstrahlung von Mikrowellenenergie in wasserhältige oder mit Wasser versetzte Materie, mit einem Hohlraumresonator (2), welcher mit zumindest einem, in seiner Energieabgabe regelbaren Mikrowellengenerator (6) in Verbindung steht, mit einem für Mikrowellen durchlässigen, gasdicht verschließbaren Gefäß (1) zur Aufnahme der zu behandelnden Materie, dessen Innenraum (3) der Hohlraumresonator (2) mit bestand umschließt, wobei der Innenraum (3) des Gefäßes (1) mit einer Druckmesseinrichtung (4) in Verbindung steht und eine die Energieabgabe des Mikrowellengenerators (6) beeinflussende, mit der Druckmeßeinrichtung (4) in Verbindung stehende Regeleinrichtung (10) vorgesehen ist, **dadurch gekennzeichnet,** daß sich das gasdicht verschließbare Gefäß (1) am Hohlraumresonator (2) abstützt und als Verkleidung des Hohlraumresonators (2) ausgebildet ist, daß eine mit einem Ventil (12) versehene Zuleitung (11) für Wasser oder Wasserdampf mit dem Innenraum (3) des Gefäßes (1) in Verbindung steht, sowie daß die zur Erreichung und Konstanthaltung eines vorgebbaren, über dem Atmosphärendruck liegenden Innendruckes benötigte Energieabgabe des Mikrowellengenerators (6) über die Regeleinrichtung (10) regelbar ist.

2. Vorrichtung nach Anspruch 1, **dadurch gekennzeichnet,** daß eine mit einem Ventil (9) abtrennbare, außerhalb des Hohlraumresonators (2) angeordnete Vakuumeinrichtung (7) zur Evakuierung des Innenraumes (3) vorgesehen ist.

3. Vorrichtung nach Anspruch 1 oder 2, **dadurch gekennzeichnet,** daß das als Verkleidung des Hohlraumresonators (2) ausgebildete Gefäß (1) aus Polytetraflourethylen besteht.

4. Vorrichtung nach einem der Ansprüche 1 bis 3, **dadurch gekennzeichnet,** daß eine mit dem Innenraum (3) des Gefäßes (1) in Verbindung stehende Temperaturmeßeinrichtung vorgesehen ist.

5. Vorrichtung nach einem der Ansprüche 1 bis 4, **dadurch gekennzeichnet,** daß die Wandstärke des gasdichten Gefäßes (1) den nötigen bestand des Innenraumes (3) von der Wand des Hohlraumresonators (2) bestimmt, welcher für eine Mikrowellenfrequenz von 2450 MHz im Bereich von 3 cm liegt.

6. Vorrichtung nach einem der Ansprüche 1 bis 5, **dadurch gekennzeichnet,** daß das Gefäß (1) und der Hohlraumresonator (2) über einen gemeinsamen Deckel (13) verfügen, welcher einen Verriegelungsmechanismus (14) mit einer Kontroll- und Sicherheitseinrichtung (15 bis 18) aufweist.

7. Verfahren zur Einstrahlung von Mikrowellenenergie in wasserhältige oder mit Wasser versetzte Materie, **gekennzeichnet durch** folgende Schritte:
a) Einbringen der zu behandelnden Materie in einen gasdichten, druckfesten, für Mikrowellenstrahlung durchlässigen Behälter,
b) Evakuieren des gasdichten Behälters,
c) Zusetzen von Wasser oder Wasserdampf in Abhängigkeit vom Wassergehalt der zu behandelnden Materie,
d) Einstrahlen von Mikrowellenenergie zur Erhitzung der Materie und des zugesetzen Wassers bzw. Wasserdampfes auf Temperaturen über 100°C und Erhöhung des Innendruckes auf einen vorgebbaren Druck über dem Atmosphärendruck,
e) Messen des Innendruckes,
f) Regeln der Mikrowelleneinstrahlung zur Erreichung und Konstanthaltung des vorgebbaren Innendruckes in Abhängigkeit des in Schritt e) erzielten Meßwertes und
g) Aufrechterhalten des in Schritt f) erreichten Innendruckes für einen vorgebbaren Zeitintervall um einen Temperaturausgleich zwischen den Materiebestandteilen zu erreichen.

## Claims

1. An apparatus for radiating microwave energy into material containing water or mixed with water, comprising a resonant cavity (2) which is connected to one or more microwave generators (6) with variable energy output, and further comprising a sealed, gas-tight container (1) which is transparent to microwaves and contains the material to be treated, whose interior (3) is enclosed by the resonant cavity (2) at a given distance, the interior (3) of the container (1) being connected with a pressure sensing device (4), and a control unit (10) being provided for control of the energy output of the microwave generator (6), which is connected to the pressure sensing device (4), **characterized in that** the gas-tight, sealable container (1) is supported in the resonant cavity (2) and is configured as a lining of the cavity (2), and that a feed line (11) for water or water vapour, which is provided with a valve (12), is connected with the interior (3) of the container (1), and further that the energy output of the microwave generator (6) required for obtaining and keeping constant a preset interior pressure greater than atmospheric, is controlled by the control unit.

2. An apparatus according to claim 1, **characterized in that** a vacuum unit (7) is provided for evacuation of the interior (3), which unit (7) is locked by a valve (9) and is placed outside of the resonant cavity (2).

3. An apparatus according to claim 1 or 2,
**characterized in that** the container (1) configured as a lining of the resonant cavity (2) is made of polytetrafluoroethylene.

4. An apparatus according to any of claims 1 to 3,
**characterized in that** a temperature sensing device is provided which is connected with the interior (3) of the container (1).

5. An apparatus according to any of claims 1 to 4,
**characterized in that** the wall thickness of the gas-tight container (1) will define the necessary distance of the interior (3) from the wall of the resonant cavity (2), which is in the range of 3 cm for a microwave frequency of 2,450 MHZ.

6. An apparatus according to any of claims 1 to 5,
**characterized in that** the container (1) and the resonant cavity (2) have a common lid (13) which is provided with a locking mechanism (14) comprising control and safety elements (15 to 18).

7. A method of the invention for radiating microwave energy into material containing water or mixed with water, **characterized by** the following steps:
(a) introduction of the material to be treated into a gas-tight, pressure-tight container transparent to microwave radiation;
(b) evacuation of the gas-tight container;
(c) addition of water or water vapour, depending on the water content of the material to be treated;
(d) radiation of microwave energy into the material, thereby heating it, and the water or water vapour added thereto, to more than 100°C and raising the interior pressure to a preset level above atmospheric pressure;
(e) determination of the interior pressure;
(f) control of the microwave radiation to obtain and keep constant the preset interior pressure in accordance with the value obtained in step (e);
(g) maintaining of the interior pressure obtained in step (f) for a given period of time to establish a uniform temperature distribution between material components.

## Revendications

1. Dispositif pour injecter de l'énergie par micro-ondes dans un produit contenant de l'eau ou déplacé avec de l'eau, comprenant un résonateur à cavité (2) coopérant avec au moins un générateur de micro-ondes (6) dont la puissance de sortie est réglable, un récipient (1) susceptible d'être fermé de manière étanche aux gaz, transparent aux micro-ondes, pour recevoir le produit à traiter, la cavité (3) entourant le résonateur (2) avec une certaine distance, la cavité (3) du récipient (1) coopérant avec un dispositif de mesure de pression (4) et une installation de régulation (10) prévue pour influencer l'émission d'énergie du générateur à micro-ondes (6) en coopérant avec une installation de régulation (10) reliée à l'installation de mesure de pression (4), dispositif caractérisé en ce que le récipient (1), fermé de manière étanche aux gaz, s'appuie sur le résonateur à cavité (2) et constitue le revêtement du résonateur à cavité (2), une conduite d'alimentation (11) équipée d'une vanne (12) pour l'eau ou la vapeur d'eau communiquant avec la cavité (3) du récipient (1) et l'énergie fournie par le générateur à micro-ondes (6), nécessaire pour atteindre et conserver une pression intérieure prédéterminée, supérieure à la pression atmosphérique, peut être régulée par l'installation de régulation (10).

2. Dispositif selon la revendication 1, caractérisé par une installation de vide (7) prévue à l'extérieur du résonateur à cavité (2), et qui peut être coupée par une vanne (9) pour faire le vide dans la cavité (3).

3. Dispositif selon la revendication 1 ou 2, caractérisé en ce que le récipient (1) servant de revêtement au résonateur à cavité (2) est en polytétrafluoréthylène.

4. Dispositif selon l'une des revendications 1 à 3, caractérisé par une installation de mesure de température qui est reliée à la cavité (3) du récipient (1).

5. Dispositif selon l'une des revendications 1 à 4, caractérisé en ce que l'épaisseur de la paroi du récipient (1), étanche aux gaz, définit l'écartement nécessaire de la cavité intérieure (3) par rapport à la paroi du résonateur à cavité (2), et qui est de l'ordre de 3 cm pour une fréquence de micro-ondes de 2450 MHz.

6. Dispositif selon l'une des revendications 1 à 5, caractérisé en ce que le récipient (1) et le résonateur à cavité (2) ont un couvercle (13) commun qui comporte un mécanisme de verrouillage (14) avec une installation de contrôle et de sécurité (15-18).

7. Procédé pour injecter de l'énergie de micro-ondes dans un produit contenant de l'eau ou déplacé avec de l'eau, caractérisé par les étapes suivantes :
a) on introduit le produit à traiter dans un récipient étanche aux gaz, résistant à la pression, transparent aux rayonnements à micro-ondes,
b) on fait le vide dans le récipient étanche aux gaz,
c) on ajoute de l'eau ou de la vapeur d'eau en fonction de la teneur en eau du produit à traiter,
d) on injecte de l'énergie à micro-ondes pour chauffer le produit et l'eau ou la vapeur d'eau ajoutées à des températures supérieures à 100°C et on augmente la pression intérieure à une pression prédéterminée supérieure à la pression atmosphérique,
e) on mesure la pression intérieure,
f) on règle le rayonnement à micro-ondes pour atteindre et maintenir constante la pression interne prédéterminée en fonction de la valeur de mesure obtenue à l'étape e) et on maintient la pression intérieure obtenue à l'étape f) pendant un intervalle de temps prédéterminé pour arriver à une compensation de température entre les composants du produit.
